# EUROPEAN PATENT SPECIFICATION

(11) **EP 3 268 493 B1**
(45) Date of publication and mention of the grant of the patent: **05.01.2022**
(21) Application number: 16713202.6
(22) Date of filing: 11.03.2016
(51) Int. Cl.: C12Q 1/6886

(54) **WHOLE BLOOD BASED MRNA MARKERS FOR PREDICTING PROSTATE CANCER AND METHODS OF DETECTING THE SAME**
VOLLBLUTBASIERTE MRNA-MARKER ZUR VORHERSAGE VON PROSTATAKREBS UND VERFAHREN ZUM NACHWEIS DAVON
MARQUEURS D'ARNM À BASE DE SANG TOTAL POUR PRÉDIRE UN CANCER DE LA PROSTATE, ET LEURS PROCÉDÉS DE DÉTECTION

(30) Priority: 12.03.2015 US 201562132064 P
(43) Date of publication of application: 17.01.2018
(73) Proprietor: Janssen Pharmaceutica NV, 2340 Beerse (BE)
(72) Inventor: RICCI, Deborah, Raritan, New Jersey 08869 (US); GORMLEY, Michael, Spring House, Pennsylvania 19477 (US); THOMAS, Shibu, Spring House, Pennsylvania 19477 (US); RAJPUROHIT, Yashoda, Spring House, Pennsylvania 19477 (US); SCHAFFER, Michael, Spring House, Pennsylvania 19477 (US)
(74) Representative: Carpmaels & Ransford LLP
(86) International application number: PCT/US2016/022004
(87) International publication number: WO 2016/145308

(56) References cited:
- WO-A1-2014/028925
- WO-A2-2009/128936
- WO-A2-2009/128936
- WO-A2-2014/151290
- WO-A2-2014/151290
- ZHIYONG GUO ET AL: "A Novel Androgen Receptor Splice Variant Is Up-regulated during Prostate Cancer Progression and Promotes Androgen Depletion?Resistant Growth", CANCER RESEARCH, AMERICAN ASSOCIATION FOR CANCER RESEARCH, US, vol. 69, no. 6, 24 February 2009 (2009-02-24), pages 2305-2313, XP008154820, ISSN: 0008-5472, DOI: 10.1158/0008-5472.CAN-08-3795 [retrieved on 2009-02-24]
- EDWIN E REYES ET AL: "Quantitative characterization of androgen receptor protein expression and cellular localization in circulating tumor cells from patients with metastatic castration-resistant prostate cancer", JOURNAL OF TRANSLATIONAL MEDICINE, BIOMED CENTRAL, vol. 12, no. 313, 26 November 2014 (2014-11-26), pages 1-15, XP021206180, ISSN: 1479-5876, DOI: 10.1186/S12967-014-0313-Z
- CYNTHIA A. HEINLEIN ET AL: "Androgen Receptor in Prostate Cancer", ENDOCRINE REVIEWS, vol. 25, no. 2, 1 April 2004 (2004-04-01), pages 276-308, XP055541038, US ISSN: 0163-769X, DOI: 10.1210/er.2002-0032
- MH EILEEN TAN ET AL: "Androgen receptor: structure, role in prostate cancer and drug discovery", ACTA PHARMACOLOGICA SINICA, vol. 36, no. 1, 1 January 2015 (2015-01-01), pages 3-23, XP055541034, GB ISSN: 1671-4083, DOI: 10.1038/aps.2014.18
- R W ROSS ET AL: "Sensitivity and specificity of a whole-blood RNA transcript-based diagnostic test for the diagnosis of prostate cancer (CaP) compared with prostate-specific antigen (PSA) alone", 2009 ASCO ANNUAL M EETING, 1 January 2009 (2009-01-01), XP055284520,
- DAVID OLMOS ET AL: "Prognostic value of blood mRNA expression signatures in castration-resistant prostate cancer: a prospective, two-stage study", LANCET ONCOLOGY, vol. 13, no. 11, 1 November 2012 (2012-11-01), page 1114, XP055284539, GB ISSN: 1470-2045, DOI: 10.1016/S1470-2045(12)70372-8
- ZHIYONG GUO ET AL: "A Novel Androgen Receptor Splice Variant Is Up-regulated during Prostate Cancer Progression and Promotes Androgen Depletion?Resistant Growth", CANCER RESEARCH, AMERICAN ASSOCIATION FOR CANCER RESEARCH, US, vol. 69, no. 6, 24 February 2009 (2009-02-24), pages 2305-2313, XP008154820, ISSN: 0008-5472, DOI: 10.1158/0008-5472.CAN-08-3795 [retrieved on 2009-02-24]
- EDWIN E REYES ET AL: "Quantitative characterization of androgen receptor protein expression and cellular localization in circulating tumor cells from patients with metastatic castration-resistant prostate cancer", 26 November 2014 (2014-11-26), JOURNAL OF TRANSLATIONAL MEDICINE, BIOMED CENTRAL, PAGE(S) 1 - 15, XP021206180, ISSN: 1479-5876
- CYNTHIA A. HEINLEIN ET AL: "Androgen Receptor in Prostate Cancer", ENDOCRINE REVIEWS, vol. 25, no. 2, 1 April 2004 (2004-04-01), pages 276-308, XP055541038, US ISSN: 0163-769X, DOI: 10.1210/er.2002-0032
- MH EILEEN TAN ET AL: "Androgen receptor: structure, role in prostate cancer and drug discovery", ACTA PHARMACOLOGICA SINICA, vol. 36, no. 1, 1 January 2015 (2015-01-01), pages 3-23, XP055541034, GB ISSN: 1671-4083, DOI: 10.1038/aps.2014.18
- Thea Grindstad ET AL: "High Progesterone Receptor Expression in Prostate Cancer Is Associated with Clinical Failure", PLOS ONE, vol. 10, no. 2, 27 February 2015 (2015-02-27), page e0116691, XP055659631, DOI: 10.1371/journal.pone.0116691
- Kathryn L. Penney ET AL: "mRNA Expression Signature of Gleason Grade Predicts Lethal Prostate Cancer", JOURNAL OF CLINICAL ONCOLOGY, vol. 29, no. 17, 10 June 2011 (2011-06-10) , pages 2391-2396, XP055390709, US ISSN: 0732-183X, DOI: 10.1200/JCO.2010.32.6421
- Laia Agell ET AL: "A 12-Gene Expression Signature Is Associated with Aggressive Histological in Prostate Cancer", AMERICAN JOURNAL OF PATHOLOGY., vol. 181, no. 5, 1 November 2012 (2012-11-01), pages 1585-1594, XP055254671, US ISSN: 0002-9440, DOI: 10.1016/j.ajpath.2012.08.005
- EDWARDS S ET AL: "Expression analysis onto microarrays of randomly selected cDNA clones highlights HOXB13 as a marker of human prostate cancer", BRITISH JOURNAL OF CANCER, NATURE PUBLISHING GROUP, GB, vol. 92, no. 2, 31 January 2005 (2005-01-31), pages 376-381, XP002341218, ISSN: 0007-0920, DOI: 10.1038/SJ.BJC.6602261

## Description

### TECHNICAL FIELD

Disclosed herein are whole blood based mRNA biomarkers for predicting prostate cancer and methods of detecting the same. In particular, the disclosed mRNA markers and methods enable the detection of prostate cancer-specific mRNA biomarkers in a whole blood sample from a patient, identification of a patient with prostate cancer, identification of a patient with high-risk prostate cancer, and treatment of a patient with prostate cancer.

### BACKGROUND

Prostate cancer is the second most common cancer among men in the United States. It is also one of the leading causes of cancer death among men of all races and Hispanic origin populations. In 2010, 196,038 men in the United States were diagnosed with prostate cancer while 28,560 men in the United States died from prostate cancer. (U.S. Cancer Statistics Working Group. United States Cancer Statistics: 1999-2010 Incidence and Mortality Web-based Report. Atlanta (GA): Department of Health and Human Services, Centers for Disease Control and Prevention, and National Cancer Institute; 2013.)

One of the major challenges in management of prostate cancer is the lack of tests to distinguish between those patients who should be treated adequately to stop the aggressive form of the disease and those who should avoid overtreatment of the indolent form. Molecular heterogeneity of prostate cancer and difficulty in acquiring tumor tissue from patients makes individualized management of prostate cancer difficult.

WO 2014/028925 relates to gene expression profiles associated with prostate cancer.

Ross et al. 2009 ASCO Annual Meeting, 1 January 2009 relates to a whole-blood RNA transcript-based diagnostic test for the diagnosis of prostate cancer.

Olmos et al. (2012) Lancet Oncology, 12:11 page 1114 relates to prognostic value of blood mRNA expression signatures in castration-resistant prostate cancer.

### SUMMARY

Provided herein is a method of detecting prostate cancer specific mRNA biomarkers in a whole blood sample from a patient comprising:
isolating RNA from the whole blood sample;
synthesizing cDNA from the isolated RNA; and
measuring an expression level of KLK3, ACADL, GRHL2, HOXB13, HSD3B1, TMP.ERG, ARV7, ARV567, FOLH1, KLK2, HSD3B2, AGR2, AZGP1, STEAP2, KCNN2, GPX8, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3, SNAI2, CDH12, NKX3.1, LGR5, TRPM8, and SLCO1B3.

In some embodiments of the method of the invention, the cDNA is preamplified after the synthesizing step.

In a further aspect of the method of the invention, the cDNA is preamplified for 14 cycles; and/or the cDNA is preamplified by qRT-PCR.

In some embodiments of the method of the invention, the method further comprises identifying high-risk prostate cancer if an increased expression level of KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2, or any combination thereof is detected.

In some embodiments of the method of the invention, increased expression of KLK3, PGR, KCNN2, MYBPC1 and HOXB13 indicates high-risk prostate cancer.

In some embodiments of the method of the invention, the method further comprises comparing the expression level of ARV7 from the patient's whole blood sample to a reference level of ARV7 expression, optionally wherein the reference level of ARV7 is an expression level of ARV7 in a whole blood sample from an individual without prostate cancer; and/or determining if the expression level of ARV7 from the patient's whole blood sample is increased or decreased relative to the reference level of ARV7 expression.

In some embodiments of the method of the invention the whole blood sample is collected in a blood collection tube.

Also provided herein is use of a gene chip comprising primer pairs and a panel of probes configured to amplify and detect KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2, or any combination thereof, for the method of the invention.

In some embodiments of the use of the invention, the gene chip comprises primer pairs and a panel of probes configured to amplify and detect KLK3, ACADL, GRHL2, HOXB13, HSD3B1, TMP.ERG, ARV7, ARV567, FOLH1, KLK2, HSD3B2, AGR2, AZGP1, STEAP2, KCNN2, GPX8, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3, SNAI2, CDH12, NKX3.1, LGR5, TRPM8 and SLCO1B3.

Also provided herein is a method of identifying a patient with high-risk prostate cancer comprising:
obtaining cDNA from a whole blood sample of the patient;
contacting the cDNA with a gene chip, wherein the gene chip comprises a set of primer pairs and a panel of probes specific for mRNA biomarkers indicative of high-risk prostate cancer, wherein the mRNA biomarkers are selected from the group consisting of KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, and GRHL2;
and measuring expression levels of all of the mRNA biomarkers; and
wherein an increase in the expression level of at least eight of the mRNA biomarkers compared to a reference level of the at least eight mRNA biomarkers is measured.

Disclosed herein are methods of detecting prostate cancer-specific mRNA biomarkers in a whole blood sample from a patient. The methods comprise, consist of and/or consist essentially of: isolating RNA from the whole blood sample; synthesizing cDNA from the isolated RNA; and measuring an expression level of at least one mRNA biomarker, wherein the at least one mRNA biomarker is or is selected from the group consisting essentially of KLK3, ACADL, GRHL2, HOXB13, HSD3B1, TMP.ERG, ARv7( ARV3.7), ARV567, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3. SNAI2, CDH12, NKX3.1, LGR5, TRPM8, SLCO1B3 and/or any combination thereof.

Methods for detecting ARv7(ARV3.7)in a whole blood sample from a patient are also disclosed. The methods comprise, consist of, and/or consist essentially of isolating RNA from the whole blood sample, synthesizing cDNA from the isolated RNA, and measuring an expression level of ARv7(ARV3.7).

Also disclosed are methods of identifying a patient with prostate cancer comprising, consisting of and/or consisting essentially of obtaining cDNA from a whole blood sample of the patient; contacting the cDNA with a gene chip, wherein the gene chip comprises a primer pair and a probe for COL1A1; measuring an expression level of COL1A1; and comparing the expression level of COL1A1 to a reference level of COL1A1, wherein an increase in the expression level of COL1A1 in the whole blood sample compared to the reference level is indicative of prostate cancer.

Methods of identifying a patient with high-risk prostate cancer are also disclosed. The methods comprise, consist of, and/or consist essentially of obtaining cDNA from a whole blood sample of the patient; contacting the cDNA with a gene chip, wherein the gene chip comprises a primer pair and a probe for at least one mRNA biomarker indicative of high-risk prostate cancer, wherein the at least one mRNA biomarker comprises, consists of and/or consists essentially of a member selected from the group consisting of KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 and/or any combination thereof; measuring an expression level of the at least one mRNA biomarker; and comparing the expression level of the at least one mRNA biomarker to a reference level of the at least one mRNA biomarker, wherein an increase in the expression level of the at least one mRNA biomarker compared to the reference level indicates high-risk prostate cancer. In addition, mRNA biomarkers may be combined into a biomarker panel including multiple mRNA biomarkers. A subject would be called biomarker positive if greater than or equal to a predetermined, e.g., 3, 4, 5, 6, 7, 8, or 9, were detected. Biomarker positive status indicates high-risk prostate cancer.

Further disclosed are methods of treating a patient with prostate cancer comprising, consisting or, and/or consisting essentially of obtaining cDNA from a whole blood sample of the patient; contacting the cDNA with a gene chip, wherein the gene chip comprises a primer pair and a probe for COL1A1; measuring an expression level of COL1A1; comparing the expression level of COL1A1 to a reference level of COL1A1; and treating the patient for prostate cancer if the expression level of COL1A1 is increased compared to the reference level of COL1A1. This example is not meant to be limiting, for example expression of mRNA biomarkers or biomarker positive status described herein may also be used to select patients for treatment.

Also disclosed are gene chips for detecting prostate cancer specific mRNA transcripts in a whole blood sample from a patient, comprising, consisting of, and/or consisting essentially of a primer pair and a probe configured to amplify and detect a member selected from the group consisting of KLK3, ACADL, GRHL2, HOXB13, HSD3B1, TMP.ERG, ARV3.7, ARV567, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3. SNAI2, CDH12, NKX3.1, LGR5, TRPM8, SLCO1B3 and/or any combination thereof.

### BRIEF DESCRIPTION OF THE DRAWINGS

The summary, as well as the following detailed description, is further understood when read in conjunction with the appended drawings. For the purpose of illustrating the disclosed methods and gene chips, there are shown in the drawings exemplary embodiments of the methods and gene chips; however, the methods and gene chips are not limited to the specific embodiments disclosed. In the drawings:

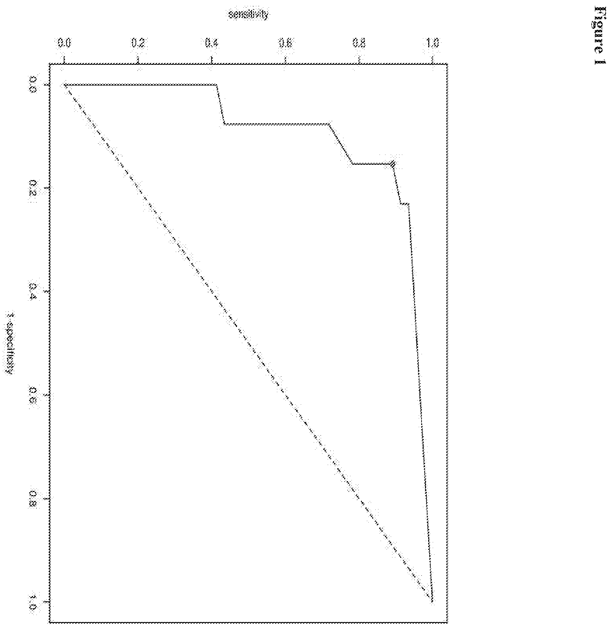

**FIG 1** represents an exemplary ROC curve for COL1A1 showing the trade-off between sensitivity and specificity at all levels of marker expression. The point indicates the optimal cutpoint which maximizes sensitivity and specificity.
**FIG 2** represents an exemplary Kaplan-Meier curve for a biomarker or biomarker panel showing the proportion of subjects that have not experienced an event such as disease recurrence or death from disease by a specific time represented on the x-axis. Biomarker positive and biomarker negative subjects are represented by the red and black lines respectively. Biomarker positive subjects have a significantly higher likelihood of experiencing an event earlier than biomarker negative subjects, i.e. biomarker positive subjects have poorer prognosis.

### DETAILED DESCRIPTION OF ILLUSTRATIVE EMBODIMENTS

The disclosed methods and gene chips may be understood more readily by reference to the following detailed description taken in connection with the accompanying figures, which form a part of this disclosure. It is to be understood that the disclosed methods and gene chips are not limited to the specific methods and gene chips described and/or shown herein, and that the terminology used herein is for the purpose of describing particular embodiments by way of example only and is not intended to be limiting of the claimed methods and gene chips.

Unless specifically stated otherwise, any description as to a possible mechanism or mode of action or reason for improvement is meant to be illustrative only, and the disclosed methods are not to be constrained by the correctness or incorrectness of any such suggested mechanism or mode of action or reason for improvement.

Reference to a particular numerical value includes at least that particular value, unless the context clearly dictates otherwise. When a range of values is expressed, another embodiment includes from the one particular value and/or to the other particular value. Further, reference to values stated in ranges include each and every value within that range. All ranges are inclusive and combinable.

When values are expressed as approximations, by use of the antecedent "about," it will be understood that the particular value forms another embodiment.

It is to be appreciated that certain features of the disclosed methods and gene chips which are, for clarity, described herein in the context of separate embodiments, may also be provided in combination in a single embodiment. Conversely, various features of the disclosed methods and gene chips that are, for brevity, described in the context of a single embodiment, may also be provided separately or in any subcombination.

As used herein, the singular forms "a," "an," and "the" include the plural.

As used herein, the term "patient" refers to any mammal whose whole blood samples can be analyzed with the disclosed methods. Thus, the disclosed methods are applicable to human and nonhuman subjects, although it is most preferably used for humans. In some embodiments, the patient sample is a human sample. In other embodiments, the patient sample is a nonhuman sample. "Patient" and "subject" may be used interchangeably herein.

As used herein, the phrase "contacting cDNA with a gene chip" refers to a procedure whereby cDNA obtained from a whole blood sample of the patient is incubated with, or added to, a gene chip in order to evaluate gene expression.

The phrase "increase in the expression level" encompasses both the presence of the mRNA biomarker and an elevated level of the mRNA biomarker relative to a reference sample. For example, one or more of the disclosed mRNA biomarkers may be absent from a reference sample. For such mRNA biomarkers, the term "increase in the expression level" refers to the presence of the mRNA biomarker in the patient's whole blood sample. Conversely, one or more of the disclosed mRNA biomarkers may be present at some level in a reference sample. For such mRNA biomarkers, the term "increase in the expression level" refers to an elevated level of the mRNA biomarker in the patient's whole blood sample compared to the reference sample.

As used herein, "reference sample" refers to a whole blood sample from an individual or population of individuals that does not have, and did not in the past have, prostate cancer. Accordingly, "reference level of" refers to the level of expression of the one or more disclosed biomarkers in a whole blood sample from an individual or population of individuals that does not have, and did not in the past have, prostate cancer.

As used herein, "a primer pair" refers to a forward primer and a reverse primer for amplifying the cDNA of the mRNA biomarker of interest.

### Detection of prostate cancer specific mRNA biomarkers

Disclosed herein are methods of detecting prostate cancer specific mRNA biomarkers in a whole blood sample from a patient. The methods comprise: isolating RNA from the whole blood sample; synthesizing cDNA from the isolated RNA; optionally, preamplifying the cDNA, and measuring an expression level of at least one mRNA biomarker, wherein the at least one mRNA biomarker is KLK3, ACADL, GRHL2, HOXB13, HSD3B1, TMP.ERG, ARV7( ARV3.7) (also known as ARV7), ARV567, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3. SNAI2, CDH12, NKX3.1, LGR5, TRPM8, SLCO1B3, or any combination thereof.

The disclosed methods enable the detection of prostate cancer specific mRNA biomarkers in a whole blood sample. Detection of these markers can be used for identifying/diagnosing a patient with prostate cancer, identifying a patient with/predicting high-risk prostate cancer, and treating a patient with prostate cancer.

As used herein, the phrase "prostate cancer specific mRNA biomarker" refers to single mRNA biomarkers or mRNA biomarker groups (i.e., two or more associated biomarkers) which may be used to detect prostate cancer from a whole blood sample. Exemplary mRNA biomarkers are listed in Table 1 and include, but are not limited to, KLK3, ACADL, GRHL2, HOXB13, HSD3B1, TMP.ERG, ARV3.7, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, BRS3. SNAI2, CDH12, NKX3.1, LGR5, TRPM8, SLCO1B3 and CYP3A5.

**Table 1 - Exemplary mRNA biomarkers**

| **mRNA Biomarker** | **Name** | **GenBank Accession No.** |
|---|---|---|
| KLK3 | kallikrein-3 | NM_001030047 |
| ACADL | Acyl-CoA Dehydrogenase, Long Chain | NM_001608.3 |
| GRHL2 | grainyhead-like 2 (Drosophila) | NM_024915.3 |
| HOXB 13 | homeobox B13 | NM_006361.5 |
| HSD3B1 | hydroxy-delta-5-steroid dehydrogenase, 3 beta- and steroid delta-isomerase 1 | NM_000862.2 |
| TMP.ERG | TMP-ERG fusion (as described in Yu. J., et al. An integrated network of androgen receptor, polycomb, and TMPRSS2-ERG gene fusions in prostate cancer progression. Cancer Cell (2010) 17(5): 443-454. | |
| ARV3.7 | Androgen receptor splice variant (as described in Hu, R., et al. Ligand-independent androgen receptor variants derived from splicing of cryptic exons signify hormone refractory prostate cancer. Cancer Res. (2009) 69(1): 16-22. | |
| FOLH1 | folate hydrolase (prostate-specific membrane antigen) 1 | NM_001193471.1 |
| KLK2 | kallikrein-related peptidase 2 | NM_001002231.2 |
| HSD3B2 | hydroxy-delta-5-steroid dehydrogenase, 3 beta- and steroid delta-isomerase 2 | NM_000198.3 |
| AGR2 | anterior gradient 2 | NM_006408.3 |
| AZGP1 | alpha-2-glycoprotein 1, zinc-binding pseudogene 1 | NR_036679.1 |
| STEAP2 | STEAP family member 2, metalloreductase | NM_001040665.1 |
| KCNN2 | potassium intermediate/small conductance calcium-activated channel, subfamily N, member 2 | NM_001278204.1 |
| GPX8 | glutathione peroxidase 8 (putative) | NM_001008397.2 |
| SLCO1B3 | solute carrier organic anion transporter family, member 1B3 | NM_019844.3 |
| TMEFF2 | transmembrane protein with EGF-like and two follistatin-like domains 2 | NM_016192.2 |
| SPINK1 | serine peptidase inhibitor, Kazal type 1 | NM_003122.3 |
| SFRP4 | secreted frizzled-related protein 4 | NM_003014.3 |
| NROB1 | nuclear receptor subfamily 0, group B, member 1 | NM_000475.4 |
| FAM13C | family with sequence similarity 13, member C | NM_001001971.2 |
| HNF1A | HNF1 homeobox A | NM_000545.5 |
| CDH12 | cadherin 12, type 2 (N-cadherin 2) | NM_004061.3 |
| PGR | progesterone receptor | NM_000926.4 |
| PITX2 | paired-like homeodomain 2 | NM_000325.5 |
| MYBPC1 | myosin binding protein C, slow type | NM_001254718.1 |
| FOXA1 | forkhead box A1 | NM_004496.3 |
| SRD5A2 | steroid-5-alpha-reductase, alpha polypeptide 2 (3-oxo-5 alpha-steroid delta 4-dehydrogenase alpha 2) | NM_000348.3 |
| COL1A1 | collagen, type I, alpha 1 | NM_000088.3 |
| NPY | neuropeptide Y | NM_000905.3 |
| UGT2B 17 | UDP glucuronosyltransferase 2 family, polypeptide B17 | NM_001077.3 |
| CLUL1 | clusterin-like 1 (retinal) | NM_014410.4 |
| C9orf152 | chromosome 9 open reading frame 152 | NM_001012993.2 |
| FLNC | filamin C, gamma | NM_001127487.1 |
| GPR39 | G protein-coupled receptor 39 | NM_001508.2 |
| RELN | Reelin | NM_005045.3 |
| THBS2 | thrombospondin 2 | NM_003247.2 |
| CYP17A1 | cytochrome P450, family 17, subfamily A, polypeptide 1 | NM_000102.3 |
| CYP3A5 | cytochrome P450, family 3, subfamily A, polypeptide 5 | NM_000777.3 |

Suitable mRNA biomarkers can be functionally classified as androgen controlled group, abiraterone resistance group, neuroendocrine bypass group, or other bypass pathway. Thus, in some instances, the at least one mRNA biomarker can be a member of the androgen controlled group, abiraterone resistance group, neuroendocrine bypass group, other bypass pathway, or any combination thereof.

Expression profiling of whole blood offers several practical advantages over that of tumor tissue, including the minimally invasive nature of sample acquisition, relative ease of standardization of sampling protocols, and the ability to obtain repeated samples over time since tumor tissues are not taken as a part of standard of care. A whole blood sample can be obtained from a patient by a number of techniques known in the art including, but not limited to, venipuncture. The whole blood sample can be collected in a blood collection tube. In some embodiments, the blood collection tube can be a PAXgene^{®} brand blood RNA tube.

Techniques for isolating RNA from a whole blood sample are known in the art. Suitable commercially available kits include, for example, QIAGEN PAXgene Blood RNA Kit, the procedure of which is described in the examples section.

Techniques for synthesizing cDNA from isolated RNA are known in the art including, but are not limited to, reverse transcription of RNA.

In some embodiments, the cDNA can be pre-amplified after the synthesizing step. The preamplifying step can be performed for any suitable number of cycles. The number of cycles depends, in part, on the amount of starting cDNA and/or the amount of cDNA required to perform the amplifying step. Suitable numbers of preamplification cycles include, but are not limited to, 1 to 20 cycles. Accordingly, the preamplification step can be performed for any of 1, 2, 3, 4, 5, 6, 7, 8, 9, 10, 11, 12, 13, 14, 15, 16, 17, 18, 19, or 20 cycles. In some aspects, the preamplification step can be performed for 2 cycles. In other aspects, the preamplification step can be performed for 14 cycles. In yet other aspects, the preamplification step can be performed for more than 20 cycles.

Measuring an expression level of at least one mRNA biomarker comprises amplifying the cDNA and detecting the amplified cDNA using a gene chip, wherein the gene chip comprises a primer pair and a probe for KLK3, ACADL, GRHL2, HOXB13, HSD3B1, TMP.ERG, ARV3.7, ARV567, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3. SNAI2, CDH12, NKX3.1, LGR5, TRPM8, SLCO1B3 or any combination thereof. In some aspects the gene chip can comprise a primer pair and one probe for each mRNA biomarker to be analyzed. In other aspects, the gene chip can comprise more than one primer pair and more than one probe for each mRNA biomarker to be analyzed.

Without intending to be bound by theory, the preamplified cDNA added to the gene chip will be bound and amplified by the primer pair specific for a region within the mRNA biomarker of interest. As the cDNA is amplified, the amplified cDNA will be bound by a probe specific for a region within the mRNA biomarker of interest. Binding of the probe to the amplified cDNA will enable the detection of the amplified cDNA as the amplification process is occurring. Thus, the disclosed methods enable the detection of an expression level of the at least one mRNA biomarker at an early stage in the amplification process, allowing for enhanced sensitivity and accuracy.

The measuring step can be performed on pre-amplified or non-preamplified cDNA. Amplifying cDNA can be performed, for example, by qRT-PCR. Suitable reagents and conditions are known to those skilled in the art. qRT-PCR can be performed on a number of suitable platforms. In some aspects, for example, the qRT-PCR can be performed using Fluidigm^{™}. Accordingly, in some embodiments, the measuring step can comprise amplifying cDNA by qRT-PCR using Fluidigm^{™} Gene expression chip on a Fluidigm^{™} platform, wherein the gene expression chip comprises at least one mRNA biomarker as discussed above.

Also disclosed are methods for detecting ARV7( ARV3.7)in a whole blood sample from a patient. The disclosed methods comprise isolating RNA from the whole blood sample, synthesizing cDNA from the isolated RNA, and measuring an expression level of ARV3.7.

In some embodiments, the cDNA can be preamplified after the synthesizing step. Suitable numbers of preamplification cycles include, but are not limited to, 1 to 20 cycles. Accordingly, the preamplification step can be performed for 1, 2, 3, 4, 5, 6, 7, 8, 9, 10, 11, 12, 13, 14, 15, 16, 17, 18, 19, or 20 cycles. In some aspects, the preamplification step can be performed for 2 cycles.

The whole blood sample can be collected in a blood collection tube, such as a PAXgene^{®} blood RNA tube.

The measuring step can be performed using any one of the disclosed gene chips which comprise a primer and probe for ARV3.7. In some instances, the measuring step can be performed using a gene chip comprising a forward primer of SEQ ID NO:2 and a reverse primer of SEQ ID NO:3. In some aspects, the gene chip can further comprise a probe of SEQ ID NO:1.

The methods of detecting ARV7( ARV3.7)expression can further comprise comparing the expression level of ARV7( ARV3.7)from the patient's whole blood sample to a reference level of ARV7( ARV3.7)expression. Suitable references levels of ARV7( ARV3.7)expression include, for example, the reference level of ARV7( ARV3.7)in a whole blood sample from an individual without prostate cancer. The comparing step can be used to determine if the expression level of ARV7( ARV3.7)from the patient's whole blood sample is increased or decreased relative to the reference level of ARV7( ARV3.7)expression.

### Methods of identifying a patient with prostate cancer

Also disclosed herein are methods of identifying a patient with prostate cancer. The disclosed methods comprise: obtaining cDNA from a whole blood sample of the patient; contacting the cDNA with a gene chip, wherein the gene chip comprises a primer pair and a probe for COL1A1; measuring an expression level of COL1A1; and comparing the expression level of COL1A1 to a reference level of COL1A1, wherein an increase in the expression level of COL1A1 in the whole blood sample compared to the reference level is indicative of prostate cancer.

cDNA can be obtained from a whole blood sample by isolating RNA from the whole blood sample and synthesizing cDNA from the isolated RNA. Suitable techniques for isolating RNA and synthesizing cDNA include those disclosed above and further disclosed in the examples section. In some embodiments, the cDNA can be pre-amplified after the synthesizing step. The preamplifying step can be performed for any suitable number of cycles including, but are not limited to, 1 to 20 cycles. Accordingly, the preamplification step can be performed for 1, 2, 3, 4, 5, 6, 7, 8, 9, 10, 11, 12, 13, 14, 15, 16, 17, 18, 19, or 20 cycles. In some aspects, the preamplifying step can performed for 14 cycles.

In some instances, the gene chip further comprises a primer pair and a probe for at least one additional mRNA biomarker, wherein the at least one additional mRNA biomarker is KLK3, ACADL, GRHL2, HOXB13, HSD3B1, TMP.ERG, ARV3.7, ARV567, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, NPY, UGT2B17, CLUL1, C9orf152, FLNC, GPR39, RELN, THBS2, CYP17A1, CYP3A5, BRS3. SNAI2, CDH12, NKX3.1, LGR5, TRPM8, SLCO1B3 or any combination thereof. In instances wherein the gene chip comprises a primer pair and a probe for at least one additional mRNA biomarker, the methods further comprises: measuring an expression level of the at least one additional mRNA biomarker; and comparing the expression level of the at least one additional mRNA biomarker to a reference level of the at least one additional mRNA biomarker, wherein an increase in the expression level of the at least one additional mRNA biomarker compared to the reference level indicates prostate cancer.

In some aspects the gene chip can comprise one primer pair and one probe for each mRNA biomarker to be analyzed. In other aspects, the gene chip can comprise more than one primer pair and more than one probe for each mRNA biomarker to be analyzed.

In instances wherein the gene chip further comprises a primer pair and a probe for at least one additional mRNA biomarker, the methods comprise measuring an expression level of COL1A1 and the at least one additional mRNA biomarker, and comparing the expression level of COL1A1 and the at least one additional mRNA biomarker to a reference level of COL1A1 and the at least one additional mRNA biomarker. For example, and without intending to be limiting, in some instances the methods can comprise contacting the cDNA with a gene chip, wherein the gene chip comprises a primer pair and a probe for COL1A1 and a primer pair and a probe for MYBPC1, measuring an expression level of COLIA1 and MYBPC1, and comparing the expression level of COLIA1 and MYBPC1 to a reference level of COLIA1 and MYBPC1, wherein an increase in the expression level of COL1A1 and MYBPC1 in the whole blood sample compared to the reference level is indicative of prostate cancer.

Measuring an expression level of COL1A1 alone or in combination with at least one additional mRNA biomarker comprises amplifying the cDNA with a primer pair for COL1A1 alone or in combination with a primer for at least one additional mRNA biomarker and detecting the amplified cDNA with a probe for COL1A1 alone or in combination with a probe for at least one additional mRNA biomarker. The measuring step can be performed on pre-amplified or non-preamplified cDNA. Amplifying cDNA can be performed, for example, by qRT-PCR. Suitable reagents and conditions are known to those skilled in the art. qRT-PCR can be performed on a number of suitable platforms. In some aspects, for example, the qRT-PCR can be performed using Fluidigm^{™}. Accordingly, in some embodiments, the measuring step can comprise amplifying cDNA by qRT-PCR using Fluidigm^{™} Gene expression chip on a Fluidigm^{™} platform, wherein the gene expression chip comprises a primer for COL1A1 alone or in combination with a primer for at least one additional mRNA biomarker as discussed above.

The whole blood sample can be collected in a blood collection tube including, but not limited to, a PAX gene RNA tube.

The methods of identifying a patient with prostate cancer can further comprise confirming the expression level of the at least one mRNA biomarker by real-time PCR.

In some embodiments, the methods can further comprise assigning a risk factor to the prostate cancer, wherein an increased expression level of KLK3, PGR, KCNN2, MYBPC1, HOXB13, or any combination thereof indicates high-risk prostate cancer.

### Methods of identifying a patient with high-risk prostate cancer

Methods of identifying a patient with high-risk prostate cancer are also disclosed. The methods comprise: obtaining cDNA from a whole blood sample of the patient; contacting the cDNA with a gene chip, wherein the gene chip comprises a primer pair and a panel of mRNA biomarkers indicative of high-risk prostate cancer, wherein the panel comprises KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 or any combination thereof; measuring an expression level of the at least one mRNA biomarker; and comparing the expression level of the at least one mRNA biomarker to a reference level of the at least one mRNA biomarker, wherein an increase in the expression level of the at least one mRNA biomarker compared to the reference level indicates high-risk prostate cancer.

The disclosed methods enable the prediction of high risk prostate cancer based on the detection of KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 or any combination thereof from a whole blood sample.

cDNA can be obtained from a whole blood sample by isolating RNA from the whole blood sample and synthesizing cDNA from the isolated RNA. Suitable techniques for isolating RNA and synthesizing cDNA include those disclosed above and further disclosed in the examples section. In some embodiments, the cDNA can be pre-amplified after the synthesizing step. The preamplifying step can be performed for any suitable number of cycles. The number of cycles depends, in part, on the amount of starting cDNA and/or the amount of cDNA required to perform the amplifying step. Suitable numbers of preamplification cycles include, but are not limited to, 1 to 20 cycles. Accordingly, the preamplification step can be performed for 1, 2, 3, 4, 5, 6, 7, 8, 9, 10, 11, 12, 13, 14, 15, 16, 17, 18, 19, or 20 cycles. In some aspects, the preamplification step can be performed for 2 cycles. In other aspects, the preamplification step can be performed for 14 cycles. In yet other aspects, the preamplification step can be performed for more than 20 cycles.

Measuring an expression level of KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 or any combination thereof comprises amplifying the cDNA with a primer pair for KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 or any combination thereof, and detecting the amplified cDNA with a probe for KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 or any combination thereof. The measuring step can be performed on pre-amplified or non-preamplified cDNA. Amplifying cDNA can be performed, for example, by qRT-PCR. Suitable reagents and conditions are known to those skilled in the art. qRT-PCR can be performed on a number of suitable platforms. In some aspects, for example, the qRT-PCR can be performed using Fluidigm^{™}. Accordingly, in some embodiments, the measuring step can comprise amplifying cDNA by qRT-PCR using Fluidigm^{™} Gene expression chip on a Fluidigm^{™} platform, wherein the gene expression chip comprises a primer for KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 or any combination thereof as discussed above.

In some aspects the gene chip can comprise one primer pair and one probe for each mRNA biomarker to be analyzed. In other aspects, the gene chip can comprise more than one primer pair and more than one probe for each mRNA biomarker to be analyzed.

The whole blood sample can be collected in a blood collection tube including, but not limited to, a PAX gene RNA tube.

The methods of identifying a patient with high-risk prostate cancer can further comprise confirming the expression level of the at least one mRNA biomarker by real-time PCR.

In some embodiments, the method can entail measuring the expression level of more than one mRNA biomarker by real-time PCR and detecting greater than a threshold number of markers with positive expression. In these embodiments, patients with greater than a threshold number of markers with positive expression are deemed to be biomarker positive. Biomarker positive status is associated with increased likelihood of high risk disease.

### Methods of treating a patient with prostate cancer

Disclosed herein are methods of treating a patient with prostate cancer comprising: obtaining cDNA from a whole blood sample of the patient; contacting the cDNA with a gene chip, wherein the gene chip comprises a primer pair and a probe for COL1A1; measuring an expression level of COL1A1; comparing the expression level of COL1A1 to a reference level of COL1A1; and treating the patient if the expression level of COL1A1 is increased compared to the reference level of COL1A1.

In some instances, the gene chip further comprises a primer pair and a probe for at least one additional mRNA biomarker, wherein the at least one additional mRNA biomarker is KLK3, ACADL, GRHL2, HOXB13, HSD3B1, TMP.ERG, ARV3.7, ARV567, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, NPY, UGT2B17, CLUL1, C9orf152, FLNC, GPR39, RELN, THBS2, CYP17A1, CYP3A5, BRS3. SNAI2, CDH12, NKX3.1, LGR5, TRPM8, SLCO1B3 or any combination thereof. In instances wherein the gene chip comprises a primer pair and a probe for at least one additional mRNA biomarker, the methods further comprise: measuring an expression level of the at least one additional mRNA biomarker; comparing the expression level of the at least one additional mRNA biomarker to a reference level of the at least one additional mRNA biomarker; and treating the patient if the expression level of the at least one additional mRNA biomarker is increased compared to the reference of the at least one additional mRNA biomarker.

In other instances, the gene chip further comprises a primer pair and probe for multiple mRNA biomarkers.

In some aspects the gene chip can comprise one primer pair and one probe for each mRNA biomarker to be analyzed. In other aspects, the gene chip can comprise more than one primer pair and more than one probe for each mRNA biomarker to be analyzed.

In instances wherein the gene chip further comprises a primer pair and a probe for at least one additional mRNA biomarker, the methods of treating a patient with prostate cancer comprise measuring an expression level of COL1A1 and the at least one additional mRNA biomarker, comparing the expression level of COL1A1 and the at least one additional mRNA biomarker to a reference level of COL1A1 and the at least one additional mRNA biomarker, and treating the patient if the expression level of COL1A1 and the at least one additional mRNA biomarker is increased compared to the reference level of COL1A1 and the at least on additional mRNA biomarker. For example, and without intending to be limiting, in some instances the methods can comprise contacting the cDNA with a gene chip, wherein the gene chip comprises a primer pair and a probe for COL1A1 and a primer pair and a probe for MYBPC1, measuring an expression level of COL1A1 and MYBPC1, comparing the expression level of COL1A1 and MYBPC1 to a reference level of COL1A1 and MYBPC1, and treating the patient if the expression level of COL1A1 and MYBPC1 is increased compared to the reference level of COL1A1 and MYBPC1.

In instances wherein the gene chip further comprises a primer and a probe for multiple mRNA biomarkers, the methods of treating a patient with prostate cancer comprise measuring an expression level of multiple mRNA biomarkers, comparing the expression level of mRNA biomarkers with a reference level of expression of each mRNA biomarker, and treating the patient if greater than a threshold number of mRNA biomarkers are detected with an expression level greater than the reference level.

cDNA can be obtained from a whole blood sample by isolating RNA from the whole blood sample and synthesizing cDNA from the isolated RNA. Suitable techniques for isolating RNA and synthesizing cDNA include those disclosed above and further disclosed in the examples section. In some embodiments, the cDNA can be pre-amplified after the synthesizing step. The preamplifying step can be performed for any suitable number of cycles. The number of cycles depends, in part, on the amount of starting cDNA and/or the amount of cDNA required to perform the amplifying step. Suitable numbers of preamplification cycles include, but are not limited to, 1 to 20 cycles. Accordingly, the preamplification step can be performed for 1, 2, 3, 4, 5, 6, 7, 8, 9, 10, 11, 12, 13, 14, 15, 16, 17, 18, 19, or 20 cycles. In some aspects, the preamplification step can be performed for 2 cycles. In other aspects, the preamplification step can be performed for 14 cycles. In yet other aspects, the preamplification step can be performed for more than 20 cycles.

Measuring an expression level of COL1A1 alone or in combination with at least one additional mRNA biomarker comprises amplifying the cDNA with a primer pair for COL1A1 alone or in combination with a primer pair for at least one additional mRNA biomarker, and detecting the amplified cDNA with a probe for COL1A1 alone or in combination with a probe for at least one additional mRNA biomarker. The measuring step can be performed on pre-amplified or non-preamplified cDNA. Amplifying cDNA can be performed, for example, by qRT-PCR. Suitable reagents and conditions are known to those skilled in the art. qRT-PCR can be performed on a number of suitable platforms. In some aspects, for example, the qRT-PCR can be performed using Fluidigm^{™}. Accordingly, in some embodiments, the measuring step can comprise amplifying cDNA by qRT-PCR using Fluidigm^{™} Gene expression chip on a Fluidigm^{™} platform, wherein the gene expression chip comprises a primer for COL1A1 alone or in combination with a primer for at least one additional mRNA biomarker as discussed above.

The whole blood sample can be collected in a blood collection tube including, but not limited to, a PAX gene RNA tube.

The methods of treating a patient with prostate cancer can further comprise confirming the expression level of the at least one mRNA biomarker by real-time PCR.

Suitable compounds for treating a patient having an increased expression level of COL1A1 or COL1A1 and the at least one additional mRNA biomarker include, but are not limited to:

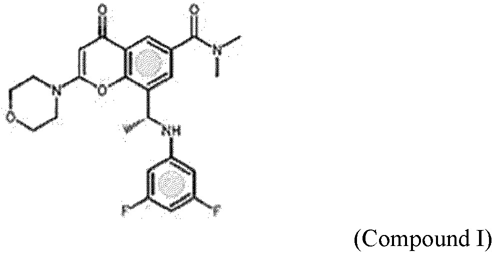

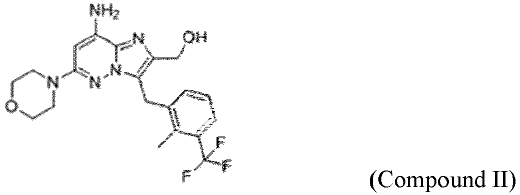

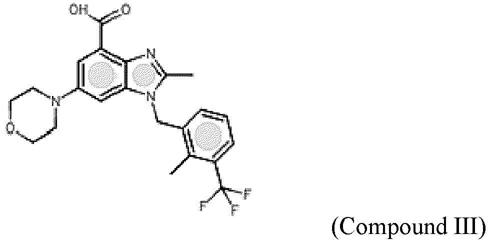

### Gene chips

Disclosed herein are gene chips for detecting prostate cancer specific mRNA transcripts in a whole blood sample from a patient. In some instances, the gene chips can comprise a primer pair and a probe configured to amplify and detect KLK3, ACADL, GRHL2, HOXB13, HSD3B1, TMP.ERG, ARV3.7, ARV567, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3. SNAI2, CDH12, NKX3.1, LGR5, TRPM8, SLCO1B3 or any combination thereof.

In some instances, the gene chips can comprise a plurality of primer pairs and a plurality of probes configured to amplify and detect KLK3, ACADL, GRHL2, HOXB13, HSD3B1, TMP.ERG, ARV3.7, ARV567, FOLH1, KLK2, HSD3B2, AGR2, AZGP1, STEAP2, KCNN2, GPX8, SLCO1B3, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3. SNAI2, CDH12, NKX3.1, LGR5, TRPM8, SLCO1B3 or any combination thereof. For example, and without intending to be limiting, in some aspects the gene chip can comprise one primer pair and one probe for each mRNA biomarker to be analyzed. In other aspects, the gene chip can comprise more than one primer pair and more than one probe for each mRNA biomarker to be analyzed.

Suitable probes include, but are not limited to, those listed in Table 2.

**Table 2 - Exemplary probes for the disclosed gene chips**

| **Assay ID (Life Technologies)** | **Target** |
|---|---|
| Hs02576345_ml | KLK3 |
| Hs00380670_ml | GPX8 |
| Hs00165843_ml | SRD5A2 |
| Hs01085277_ml | ACADL |
| Hs00227745_ml | GRHL2 |
| H00426435_ml | HSD3B1 |
| Hs00197189_ml | HOXB13 |
| Hs00428383_ml | KLK2 |
| Hs03043658_ml | NROB1 |
| Hs00167041_ml | HNF1A |
| Hs01591157_ml | AZGP1P1 |
| Hs00189528_ml | FOLH1 (PSMA) |
| Hs04234069_mH | PTX2(PITX2) |
| Hs00300346_ml | FAM13C |
| Hs00164004_ml | COL1A1 |
| Hs01556702_ml | PGR |
| Hs00362037_ml | N-Cadherin(CDH12) |
| Hs00605123_ml | HSD3B2 |
| Hs01030641_ml | KCNN2 |
| Hs00401292_ml | STEAP2 |
| Hs00180066_ml | SFRP4 |
| Hs00356521_ml | AGR2 |
| Hs00162154_ml | SPINK1 |
| Hs00270129_ml | FOXA1 |
| Hs00251986_ml | SLCO1B3 |
| Hs01086906_ml | TMEFF2 |
| Hs00159451_ml | MYBPC1 |
| Hs00179951_ml | (Bombesin)BRS3 |
| Hs00900370_ml | CHGA |
| Hs00969422_ml | LGR5 |
| Hs00950344_ml | SNAI2 |

| | |
|---|---|
| TaqMan^{®} Reporter = FAM | |

Suitable primers and probes for TMP:ERG and ARV7( ARV3.7)include, but are not limited to, those listed in Table 3.

**Table 3 - Exemplary primers and probes for the disclosed gene chips**

| **Assay ID** | **Target** | **Probe Sequences** | **Forward Primer** | **Reverse Primer 5'-3'** |
|---|---|---|---|---|
| **Androgen Receptor Assays** | | | | |
| Custom | AR Variant 3/Variant 7 (ARV3.7) | CTGGGAGAAAA ATTCCGGGT (SEQ ID NO:1) | GGAAATGTTATGA AGCAGGGATG (SEQ ID NO:2) | TTTGAGATGCTTG CAATTGCC (SEQ ID NO:3) |
| Custom | AR Variant 567 (ARV567) | CTTGCCTGATTG CGAGAG (SEQ ID NO:4) | CTGGGAGAGAGAC AGCTTGTACAC (SEQ ID NO:5) | CAGGTCAAAAGTG AACTGATGCA (SEQ ID NO:6) |
| **TMPRSS2:ET S Fusion Assays** | | | | |
| Custom | TMPRSS2: ERG (TMP:ERG) | CGGCAGGAAGCC TTAT (SEQ ID NO:7) | GAGCTAAGCAGGA GGCGGA (SEQ ID NO:8) | TAGGCACACTCAA ACAACGACTG (SEQ ID NO:9) |
| **Control Gene Assays** | | | | |
| Custom | RPL19 | CCACAAGCTGAA GGC (SEQ ID NO:10) | GCGGATTCTCATGG AACACA (SEQ ID NO:11) | GGTCAGCCAGGAG CTTCTTG (SEQ ID NO:12) |

| | | | | |
|---|---|---|---|---|
| All assays used FAM TaqMan Reporter. The control gene assays used endogenous control. | | | | |

### EXAMPLES

### METHODS

### PAXgene^{®} blood RNA manual extraction procedure

RNA was extracted using QIAGEN's PAXgene^{®} Blood RNA Kit as described in the PAXgene Blood RNA Kit Handbook (PreAnalytiX GmbH; March 2009), described briefly below.

The PAXgene^{®} Blood RNA Tube was centrifuged for 10 min at 3000-5000 x g using a swing-out rotor. The supernatant was removed by decanting or pipetting. 4 ml RNase-free water was added to the pellet, and the tube was closed using a fresh secondary BD Hemogard closure. The tube was vortexed until the pellet was visibly dissolved, and centrifuged for 10 min at 3000-5000 x g. The entire supernatant was removed and discarded.

350 µl of Buffer BR1 was added and vortexed until the pellet was visibly dissolved. The sample was pipetted into a 1.5 ml microcentrifuge tube. 300 µl Buffer BR2 and 40 µl proteinase K was added. The sample was mixed by vortexing for 5 seconds, and incubated for 10 minutes at 55°C using a shaker-incubator at 400-1400 rpm.

The lysate was pipetted directly into a PAXgene^{®} Shredder spin column (lilac) placed in a 2 ml processing tube, and centrifuged for 3 minutes at maximum speed (but not to exceed 20,000 x g). The entire supernatant of the flow-through fraction was carefully transferred to a fresh 1.5 ml microcentrifuge tube without disturbing the pellet in the processing tube. 350 µl ethanol (96-100%) was added, mixed by vortexing, and centrifuged briefly to remove drops from the inside of the tube lid.

700 µl of sample was pipetted into the PAXgene^{®} RNA spin column (red) placed in a 2 ml processing tube, and centrifuged for 1 min at 8000-20,000 x g. The spin column was placed in a new 2 ml processing tube. The remaining sample was pipetted into the PAXgene^{®} RNA spin column, and centrifuged for 1 min at 8000-20,000 x g. The spin column was placed in a new 2 ml processing tube, and the old processing tube containing flow-through was discarded.

350 µl of Buffer BR3 was pipetted into the PAXgene^{®} RNA spin column and centrifuged for 1 min at 8000-20,000 x g. The spin column was placed in a new 2 ml processing tube, and the old processing tube containing flow-through was discarded.

10 µl DNase I stock solution was added to 70 µl Buffer RDD in a 1.5 ml microcentrifuge tube and mixed gently. The DNase I incubation mix (80 µl) was pipetted directly onto the PAXgene^{®} RNA spin column membrane, and placed on the bench-top (20 - 30°C) for 15 min. 350 µl of Buffer BR3 was pipetted into the PAXgene^{®} RNA spin column, and centrifuged for 1 minute at 8,000 - 20,000 x g. The spin column was placed in a new 2 ml processing tube, and the old processing tube containing flow-through was discarded.

Another 500 µl of Buffer BR4 was added to the PAXgene RNA spin column and centrifuged for 3 minutes at 8000 - 20,000 x g. The tube containing the flow-through was discarded and the PAXgene^{®} RNA spin column was placed in a new 2 ml processing tube and centrifuged for 1 minute at 8000 - 20,000 x g.

The tube containing the flow-through was discarded. The PAXgene^{®} RNA spin column was placed in a 1.5 ml microcentrifuge tube, and 40 µl of Buffer BR5 was pipetted directly onto the PAXgene^{®} RNA spin column membrane. The column was centrifuged for 1 minute at 8000-20,000 x g to elute the RNA. This step was repeated using 40 µl of Buffer BR5 and the same microcentrifuge tube.

The eluate was incubated for 5 min at 65°C in the shaker-incubator without shaking. After the incubation, the tube was chilled immediately on ice. If the RNA samples were not used immediately, they were stored at -20°C or -70°C.

### cDNA Synthesis

Preparation of 2x reverse transcription master mix: Master Mix (2x) was prepared on ice as shown in Table 4 for the appropriate number of reactions (# reactions + 10%, per 20-µL reaction). Note: 10 µL of sample RNA was added to the master mix to have final reaction volume of 20 µL.

**Table 4**

| Component | Volume (µL) for One Reaction |
|---|---|
| 10X RT Buffer Mix | 2 |
| 25X dNTP Mix | 0.8 |
| 10X RT Random Primers | 2 |
| 50U/µL MultiScribe Reverse Transcriptase | 1 |
| RNase inhibitor | 1 |
| Nuclease/RNase free H20 | 3.2 |
| **Total volume** | **10** |

The Master Mix was vortexed several times (5 to 10) to mix, and then centrifuged briefly (1500 x g, 5 to 10 sec). 10 µl of reaction mix was added to each appropriate well of a 96-well plate.

Addition of RNA sample to master mix: 10 µL of each RNA sample was added to the appropriate well of the 96-well plate, including the water negative control, to have a final reaction volume of 20 µL. The solutions were mixed gently by pipetting up and down 3 times. The 96-well reaction plate was sealed with a plate seal and centrifuged briefly (1500 x g for 60 seconds).

ABI 9700 set up: The ABI 9700 was set up as follows:
Step 1: 25°C for 10 min
Step 2: 37°C for 120 min
Step 3: 85°C for 5 sec
Step 4: 4°C infinite hold
Reaction volume was set to 20 µL.

### cDNA preamplification

Preparation of primer probe assay mix for preamplification and real-time PCR: 100 µl of 20X Primer-Probe mixture was prepared for Real Time PCR.

Preparation of 0.2X preamp assay pool: 0.2X preamp assay pool was prepared as shown in Table 5. Note: The following volumes are for the preparation of 400 µl of 0.2X preamp assay pool. Volumes can be adjusted accordingly depending on the number of samples/Taqman assays being tested.

**Table 5**

| | | | | | | |
|---|---|---|---|---|---|---|
| **Preamp Stock** | | | | | | |

| TaqMan Assay | # of TaqMan Assays | Stock aliquot | Primer pool | 1x TE (µl) | Final Volume (µl) | Final Concentration |
|---|---|---|---|---|---|---|
| 20x concentration | 48 | 4 | 192 | 208 | 400 | 0.2x |

Sample and 2x master mix preparation: The synthesized cDNA reaction plate and 0.2X assay mix pool were placed on ice. 2X preamp master mix was prepared as shown in Table 6.

**Table 6**

| Component | Volume (µL) for One Reaction |
|---|---|
| 2X TaqMan PreAmp Master Mix | 7.5 |
| 0.2X Assay Pool 1 | 3.75 |
| **Total volume** | **11.25** |

11.25 µL of Master Mix was aliquoted into the appropriate wells of a 96-well reaction plate. 3.75 µL of each cDNA sample, including positive and negative controls, were transferred into the appropriate wells in the Master Mix reaction plate, mixed by pipetting up and down 3 times, and centrifuging briefly.

ABI 9700 set up: The ABI 9700 was set up as follows:
Step 1: 95°C for 10 min
Step 2: 95°C for 15 sec
Step 3: 60°C for 4 min
Step 4: Set Step 2-3 for 14 cycles
Step 5: 4°C infinite hold
Reaction volume was set 15 µL

Preamp product dilution: The PreAmp reaction plate was centrifuged briefly (1500 x g for 60 seconds) after preamplification was completed. 135 µl of IDTE was added to each reaction well (1:10 dilution), mixed well by pipetting up and down 3 times and centrifuged briefly (1500 x g for 5 to 10 seconds). The PreAmp product was stored at -20°C until further use.

### Fluidigm^{™} 96.96 Real-Time PCR

Preparing 10X assays: Aliquots of 10X assays were prepared using volumes in Table 7. The volumes can be scaled up appropriately for multiple runs.

**Table 7**

| Component | Volume per Inlet (µL) | Volume per Inlet with Overage (µL) |
|---|---|---|
| 20X TaqMan assay | 2.5 | 3 |
| 2X Assay Loading Reagent | 2.5 | 3 |
| Total Volume | 5.0 | 6 |

Preparing sample pre-mix and samples: The components in Table 8 were combined to make Sample Pre-Mix and final Sample Mixture. The volumes can be scaled up appropriately for multiple runs.

**Table 8**

| Component | Volume per Inlet (µL) | Volume per Inlet with Overage (µL) |
|---|---|---|
| TaqMan^{®} Genotyping Mix (2X) | 2.5 | 3 |
| 20X GE Sample Loading Reagent | 0.25 | 0.3 |
| Diluted preamp | 2.25 | 2.7 |
| Total Volume | 5.0 | 6 |

| | | |
|---|---|---|
| The TaqMan Genotyping Mix was combined with the GE Sample Loading Reagent in a 1.5 mL tube - enough volume to fill an entire chip. 3.3 µL of this Sample Pre-Mix can then be aliquoted for each sample. | | |

Loading the Chip: Upon completion of the Prime (136x) script, the primed chip was removed from the IFC Controller HX. 5 µL of each assay and each sample was pipetted into their respective inlets on the chip. The chip was returned to the IFC Controller HX. Using the IFC Controller HX software, the Load Mix (136x) script was run to load the samples and assays into the chip. When the Load Mix (136x) script was complete, the loaded chip was removed from the IFC Controller.

Using the Data Collection Software: The chip was loaded on BioMark and instructions/run 96.96 specific protocols for gene expression assay were followed.

Analysis: Auto detector was selected for data analysis. CT <35 and present even in one of 4 replicates - the sample was considered positive for amplification.

### AR Axis-Liquid Biopsy Assay development-methodology

Primer validation: Custom designs and revalidated Taqman gene expression assays were ordered from ABI (detailed list of markers and corresponding gene IDs are provided in Tables 2 and 3). A panel of 4 prostate cancer cell lines (VCaP, LNCaP, 22RV1 and PC-3) shown to express the majority of these genes were used for Assay and primer validation.

Validation on FFPET (formalin fixed paraffin embedded tissue) derived RNA: To test if these markers represent true aggressive tumors, the assays were tested on a set of 40 FFPE prostate cancer and adjacent normal tissue derived RNA samples. RNA from FFPET blocks were extracted using Qiagen's All Prep DNA/RNA FFPET Kit. RNA concentration was checked on Agilent BioAnalyzer. 350-500 ng of total RNA in 12 µl volume was reverse transcribed using Qiagen's Quantitect^{™}Reverse Transcription kit and protocol. Approximately 1/3rd of the cDNA was preamplified in a 25 µl reaction volume with 48 markers for 14 cycles using TaqMan PreAmp Master Mix/protocol. Pre amplified cDNA was diluted in a 1:20 ratio with 1x TE buffer. The diluted preamp product was loaded on 96.96 Gene Expression chip and run on Biomark following the user guide. ACTB and GAPDH were used as endogenous controls. The samples were tested in quadruplicates (described above - RNA extraction using Qiagen DNA-RNA FFPET Kit).

Cell lines spiked in PAXgene^{®} blood: To test if the markers are differentially expressed in a background of whole blood cells (WBCs), VCaP cell lines were spiked in serial dilutions (10, 50, 100 and 500 cells) into PAXgene^{®} (Quiagen, Valencia, CA) blood samples from normal donors. Total RNA was extracted using Qiagen's PAXgene^{®} Blood RNA protocol (described above). RNA concentration was measured on Agilent Bioanalyzer system. 10 µl of RNA was used for cDNA prep using Applied Biosystems High Capacity cDNA Reverse Transcription kit/protocol. Approximately 1/3 of the cDNA was preamplified in a 15 µl reaction volume with 48 gene markers for 14 cycles using TaqMan PreAmp Master Mix/protocol (Applied Biosystems). Preamplified cDNA was further diluted to 1:10 ratio with 1xTE buffer. Following Fluidigm's BioMark user guide, the diluted preamp product was loaded on 96.96 Gene Expression chip and run on Biomark. Each sample and marker was tested in duplicate, thus resulting in 4 values for each gene/sample. ACTB, GAPDH and RPL19 were used as endogenous controls and BST1 and PTPRC were used as WBC Controls.

Testing PAXgene^{®} derived RNA from Prostate cancer patients: Approximately 170 markers were tested in PAXgene^{®} RNA samples derived from 143 prostate cancer patients and 20 normal male subjects (cancer samples procured from Capital Biosciences, Cureline, and Conversant Bio). Markers that were differentially detected from aggressive versus indolent or normal donor were shortlisted.

RT-PCR assay on ViiA 7 ^{™} instrument to confirm BioMark results: Markers that were shown to be detected on Biomark^{™} (Fluidigm, San Franciso, CA) platform were further tested on ViiA7^{™} (Life Technologies, Carlsbad, CA) to validate/confirm the results. All the markers that are subsequently validated on ViiA7 will be shortlisted to make the liquid biopsy panel.

Fluidigm^{®} BioMark^{™} HD System: This high-throughput real-time PCR assay was performed using the Fluidigm^{®} BioMark^{™} HD System, which enables simultaneous detection of 96 analytes in 96 samples creating 9,216 data points from a single run. The BioMark^{™} HD platform uses microfluidic distribution of sample and assays requiring only 7 nL reactions and takes less than 3 hours to complete.

Methodology: RNA from FFPET, and PAXgene^{®} RNA samples were Reverse Transcribed using Applied Biosystems High Capacity cDNA Reverse Transcription Kit followed by pre amplification of cDNA for 10/14 cycles using Applied Biosystems Taqman Pre-Amp Master Mix. The amplified cDNA was diluted and tested on Applied Biosystems's ViiA7^{™} or the Fluidigm^{®} Biomark^{™} platform for gene expression.

### Detection of mRNA markers specifically detected from whole blood from prostate cancer patients

Data consisting of mRNA marker expression levels from whole blood collected from prostate cancer patients and healthy controls was used to derive the assay parameters. In order to allow for independent validation of assays, data was split into training and validation sets in a 70%:30% ratio. Threshold Ct values were derived based on Receiver Operating Characteristic (ROC) analysis in the training data and diagnostic characteristics of the assays were evaluated using sensitivity, specificity and area under the ROC curve (AUC). Assays were independently evaluated on the validation data, using the threshold values obtained from the training data to predict which patients have prostate cancer and evaluating the assays with sensitivity and specificity. Threshold Ct values determined for each marker are listed in Table 9. Individual ROC curves for representative markers are illustrated in **FIG. 1****.**

**Table 9 - Diagnostic information for a selected set of markers**

| Marker | Application | Cutpoint | Training | | | Validation | |
|---|---|---|---|---|---|---|---|
| | | | Sens | Spec | AUC | Sens | Spec |
| COL1A1 | Diagnostic | 30.7 | 0.891 | 0.846 | 0.904 | 0.778 | 0.875 |
| MYBPC1 | Diagnostic, High risk | 29.4 | 0.783 | 0.769 | 0.746 | 0.722 | 0.375 |
| FAM13C | Diagnostic | 29.2 | 0.674 | 0.692 | 0.719 | 0.722 | 0.5 |
| GPX8 | Diagnostic | 32.9 | 0.652 | 0.692 | 0.676 | 0.556 | 0.875 |
| HOXB 13 | Diagnostic, High risk | 31.4 | 0.457 | 0.846 | 0.648 | 0.278 | 1 |
| SFRP4 | Diagnostic | 30.1 | 0.609 | 0.769 | 0.635 | 0.5 | 0.5 |
| PITX2 | Diagnostic | 29.9 | 0.5 | 0.769 | 0.626 | 0.5 | 0.625 |
| PGR | High risk | 30.3 | 0.457 | 0.538 | 0.419 | 0.389 | 0.375 |
| KLK3 | High risk | 39.1 | 0.152 | 1 | 0.576 | 0.167 | 1 |
| KCNN2 | High risk | 36.4 | 0.652 | 0.462 | 0.492 | 0.667 | 0.75 |

Markers are represented by gene symbol. Cutpoint indicates the threshold Ct value of detection for each marker. Sensitivity (Sens) is equal to the probability that the marker will be detected in a patient with prostate cancer. Specificity is equal to the probability that the marker will not be detected in a patient without prostate cancer. The area under the ROC curve (AUC) is the probability that the marker will be expressed at a higher level in prostate cancer patients relative to healthy controls.

### Prediction of high-risk prostate cancer based on detection of mRNA markers from whole blood

The prognostic power of selected markers was evaluated in a dataset consisting of expression levels of mRNA markers in whole blood collected from prostate cancer patients and healthy controls. Threshold values were derived using ROC analysis described above to discriminate between prostate cancer and normal healthy controls in training data. Association with clinical risk factors was evaluated using the Fisher Exact test. Selected markers which are significantly associated with clinical risk factors are listed in Tables 10 - 12.

**Table 10 - Association of presence/absence of mRNA markers with Gleason score**

| **Marker** | **Expression** | **N** | **GLEASON. LOW** | **GLEASON.HI GH** | **p.val** |
|---|---|---|---|---|---|
| HOXB13 | Absent | 38 | 31 | 7 | - |
| | Present | 26 | 12 | 14 | 0.0060 |
| PGR | Absent | 36 | 19 | 17 | - |
| | Present | 28 | 24 | 4 | 0.0072 |

Marker expression was dichotomized using thresholds derived for optimal detection of prostate tumors vs. normal healthy controls. Patients were dichotomized into low (Gleason <= 6) and high (Gleason >= 8) risk subsets using Gleason scores from diagnosis. The table shows the total number of tumors with/without expression of each marker (N) and the tabulation of marker detection in Gleason Low and Gleason High risk groups. Significant association is determined by the Fisher Exact Test (p.val).

**Table 11 - Association of presence/absence of mRNA markers with PSA levels measured at diagnosis**

| **Transcript** | **Expression** | **N** | **PSA.LOW** | **PSA.HIGH** | **p.val** |
|---|---|---|---|---|---|
| KLK3 | Absent | 54 | 44 | 10 | - |
| | Present | 9 | 4 | 5 | 0.0287 |
| PGR | Absent | 35 | 23 | 12 | - |
| | Present | 28 | 25 | 3 | 0.0385 |

Marker expression was dichotomized using thresholds derived for optimal detection of prostate tumors vs. normal healthy controls. Patients were dichotomized into low (PSA < 20 ng/mL) and high (PSA >= 20 ng/mL) risk subsets. The table shows the total number of tumors with/without expression of each marker (N) and the tabulation of marker detection in PSA Low and PSA High risk groups. Significant association is determined by the Fisher Exact Test (p.val).

**Table 12 - Association of presence/absence of mRNA markers with metastasis**

| **Transcript** | **Expression** | **N** | **LOCAL** | **MET** | **p.val** |
|---|---|---|---|---|---|
| PGR | Absent | 36 | 5 | 31 | - |
| | Present | 28 | 13 | 15 | 0.0055 |
| KCNN2 | Absent | 22 | 2 | 20 | - |
| | Present | 42 | 16 | 26 | 0.0187 |
| MYBPC1 | Absent | 15 | 1 | 14 | - |
| | Present | 49 | 17 | 32 | 0.0482 |

Marker expression was dichotomized using thresholds derived for optimal detection of prostate tumors vs. normal healthy controls. The table shows the total number of tumors with/without expression of each marker (N) and the tabulation of marker detection in tumors with/without metastatic recurrence. Significant association is determined by the Fisher Exact Test (p.val).

### Identification of a multivariate biomarker panel indicative of high risk prostate cancer

A biomarker panel including multiple mRNA biomarkers was identified in a dataset consisting of expression levels of mRNA markers in whole blood collected from prostate cancer patients and healthy controls. Sixteen gene (ACADL, AGR2, COL1A1, FAM13C, GPX8, GRHL2, HNF1A, HOXB13, KLK2, KLK3, MYBPC1, NR0B1, PITX2, SFRP4, SLCO1B3, TMEFF2) were identified with significantly higher expression in prostate cancer samples relative to healthy volunteers. These 16 genes were combined into a multivariate biomarker panel as described below. Threshold values were derived using ROC analysis as described above. Thresholds were selected to identify prostate cancer patients with 90% specificity, i.e. 90% or more of healthy volunteers would be correctly identified as healthy volunteers. A machine learning process was used to define the number of detected positive biomarkers at which a patient should be deemed high risk. In order to guard against overfitting, the data was split into training and validation sets as described above. The training set is used to define the classification rule including the optimal number of positive markers. Specifically, bootstrap samples were generated by randomly selecting 100 samples from the training data. Classification rules were created by evaluating the correlation of the predicted biomarker status with the time to biochemical recurrence. Correlation was measured using the concordance index, which measures the probability that a subject with a biomarker positive state will experience biochemical recurrence prior to a subject with a biomarker negative state. Eight markers was identified as the optimal number of features for the classification rule, i.e., subjects with greater than 8 markers positive would be deemed to be biomarker positive and would be predicted to have shorter time to biochemical recurrence. The association between the classification rule and time to biochemical recurrence was validated using the independent validation set of samples using cox regression analysis. The multivariate biomarker panel identified a subset of subjects with significantly shorter time to biochemical recurrence compared to the biomarker negative population (HR = 7.94, p-value = 0.024).

## Claims

1. A method of detecting prostate cancer specific mRNA biomarkers in a whole blood sample from a patient comprising:
isolating RNA from the whole blood sample;
synthesizing cDNA from the isolated RNA; and
measuring an expression level of KLK3, ACADL, GRHL2, HOXB13, HSD3B1, TMP.ERG, ARV7, ARV567, FOLH1, KLK2, HSD3B2, AGR2, AZGP1, STEAP2, KCNN2, GPX8, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3, SNAI2, CDH12, NKX3.1, LGR5, TRPM8, and SLCO1B3.

2. The method of claim 1, wherein the cDNA is preamplified after the synthesizing step.

3. The method of claim 2, wherein the cDNA is preamplified for 14 cycles; and/or wherein the cDNA is preamplified by qRT-PCR.

4. The method of any one of claims 1 to 3, wherein the method further comprises identifying high-risk prostate cancer if an increased expression level of KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2, or any combination thereof is detected.

5. The method of claim 4, wherein increased expression of KLK3, PGR, KCNN2, MYBPC1 and HOXB13 indicates high-risk prostate cancer.

6. The method of any one of claims 1 to 3, wherein the method further comprises
comparing the expression level of ARV7 from the patient's whole blood sample to a reference level of ARV7 expression, optionally wherein the reference level of ARV7 is an expression level of ARV7 in a whole blood sample from an individual without prostate cancer; and/or
determining if the expression level of ARV7 from the patient's whole blood sample is increased or decreased relative to the reference level of ARV7 expression.

7. The method of any one of claims 1 to 6, wherein the whole blood sample is collected in a blood collection tube.

8. Use of a gene chip, comprising primer pairs and a panel of probes configured to amplify and detect KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2, or any combination thereof, for the method of any one of claims 1 to 7.

9. The use of claim 8, wherein the gene chip comprises primer pairs and a panel of probes configured to amplify and detect KLK3, ACADL, GRHL2, HOXB13, HSD3B1, TMP.ERG, ARV7, ARV567, FOLH1, KLK2, HSD3B2, AGR2, AZGP1, STEAP2, KCNN2, GPX8, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3, SNAI2, CDH12, NKX3.1, LGR5, TRPM8 and SLCO1B3.

10. A method of identifying a patient with high-risk prostate cancer comprising:
obtaining cDNA from a whole blood sample of the patient;
contacting the cDNA with a gene chip, wherein the gene chip comprises a set of primer pairs and a panel of probes specific for mRNA biomarkers indicative of high-risk prostate cancer, wherein the mRNA biomarkers are selected from the group consisting of KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, and GRHL2;
and measuring expression levels of all of the mRNA biomarkers; and
wherein an increase in the expression level of at least eight of the mRNA biomarkers compared to a reference level of the at least eight mRNA biomarkers is measured.

## Patentansprüche

1. Verfahren zum Nachweisen von Prostatakrebsspezifischen MRNA-Biomarkern in einer Vollblutprobe eines Patienten, umfassend:
Isolieren von RNA aus der Vollblutprobe;
Synthetisieren von cDNA aus der isolierten RNA; und
Messung eines Expressionspegels von KLK3, ACADL, GRHL2, HOXB13, HSD3B1, TMP.ERG, ARV7, ARV567, FOLH1, KLK2, HSD3B2, AGR2, AZGP1, STEAP2, KCNN2, GPX8, TMEFF2, SPINK1, SFRP4, NROB1, FAM13C, HNF1A, CDH12, PGR, PITX2, MYBPC1, FOXA1, SRDSA2, COL1A1, NPY, UGT2B17, CLUL1, C9orf152, FLNC, GPR39, RELN, THBS2, CYP17A1, CYP3A5, BRS3, SNAI2, CDH12, NKX3.1, LGR5, TRPM8 und SLCO1B3.

2. Verfahren nach Anspruch 1, wobei die cDNA nach dem Syntheseschritt voramplifiziert wird.

3. Verfahren nach Anspruch 2, wobei die cDNA für 14 Zyklen voramplifiziert wird; und/oder wobei die cDNA durch qRT-PCR voramplifiziert wird.

4. Verfahren nach einem der Ansprüche 1 bis 3, wobei das Verfahren ferner das Identifizieren von Hochrisiko-Prostatakrebs umfasst, wenn ein erhöhter Expressionspegel von KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 oder einer beliebigen Kombination davon nachgewiesen wird.

5. Verfahren nach Anspruch 4, wobei eine erhöhte Expression von KLK3, PGR, KCNN2, MYBPC1 und HOXB13 Hochrisiko-Prostatakrebs anzeigt.

6. Verfahren nach einem der Ansprüche 1 bis 3, wobei das Verfahren ferner umfasst:
Vergleichen des Expressionspegels von ARV7 aus der Vollblutprobe des Patienten mit einem Referenzpegel der ARV7-Expression, optional wobei der Referenzpegel von ARV7 ein Expressionspegel von ARV7 in einer Vollblutprobe eines Individuums ohne Prostatakrebs ist; und/oder
Bestimmen, ob der Expressionspegel von ARV7 aus der Vollblutprobe des Patienten relativ zum Referenzpegel der ARV7-Expression erhöht oder verringert ist.

7. Verfahren nach einem der Ansprüche 1 bis 6, wobei die Vollblutprobe in einem Blutsammelröhrchen gesammelt wird.

8. Verwendung eines Genchips, bestehend aus Primerpaaren und einer Reihe von Sonden, konfiguriert zum Amplifizieren und Detektieren von KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2 oder einer beliebigen Kombination davon für das Verfahren nach einem der Ansprüche 1 bis 7.

9. Verwendung nach Anspruch 8, wobei der Genchip Primerpaare und eine Reihe von Sonden umfasst, die dazu konfiguriert sind, KLK3, ACADL, GRHL2, HOXB13, HSD3B1, TMP.ERG, ARV7, ARV567, FOLH1, KLK2, HSD3B2, AGR2, AZGP1, STEAP2, KCNN2, GPX8, TMEFF2, SPINK1, SFRP4, NROB1, FAM13C, HNF1A, CDH12, PGR, PITX2, MYBPC1, FOXA1, SRDS5A2, COL1A1, NPY, UGT2B17, CLUL1, C9orf152, FLNC, GPR39, RELN, THBS2, CYP17A1, CYP3A5, BRS3, SNAI2, CDH12, NKX3.1, LGR5, TRPM8 und SLCO1B3 zu amplifizieren und nachzuweisen.

10. Verfahren zum Identifizieren eines Patienten mit Hochrisiko-Prostatakrebs, umfassend:
Erhalten von cDNA aus einer Vollblutprobe des Patienten;
Inberührungbringen der cDNA mit einem Genchip, wobei der Genchip einen Satz von Primerpaaren und eine Reihe von Sonden, die spezifisch für mRNA-Biomarker sind, umfasst, welche auf Hochrisiko-Prostatakrebs hinweisen, wobei die mRNA-Biomarker ausgewählt sind aus der Gruppe bestehend aus KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A und GRHL2;
und Messen der Expressionspegel aller mRNA-Biomarker; und
wobei ein Anstieg des Expressionspegels von mindestens acht der mRNA-Biomarker im Vergleich zu einem Referenzpegel der mindestens acht mRNA-Biomarker gemessen wird.

## Revendications

1. Procédé de détection de biomarqueurs d'ARNm spécifiques au cancer de la prostate dans un échantillon de sang complet d'un patient comprenant :
l'isolement d'ARN à partir de l'échantillon de sang complet ;
la synthèse d'un ADNc à partir de l'ARN isolé ; et
la mesure d'un niveau d'expression de KLK3, ACADL, GRHL2, HOXB13, HSD3B1, TMP.ERG, ARV7, ARV567, FOLH1, KLK2, HSD3B2, AGR2, AZGP1, STEAP2, KCNN2, GPX8, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3, SNAI2, CDH12, NKX3.1, LGR5, TRPM8, et SLCO1B3.

2. Procédé selon la revendication 1, l'ADNc étant préamplifié après l'étape de synthèse.

3. Procédé selon la revendication 2, l'ADNc étant préamplifié pendant 14 cycles ; et/ou l'ADNc étant préamplifié par qRT-PCR.

4. Procédé selon l'une quelconque des revendications 1 à 3, le procédé comprenant en outre l'identification d'un risque élevé de cancer de la prostate si un niveau d'expression augmenté de KLK3, PGR, KCNN2, MYBPC, HOXB13, COL1A1, GPX8, FAM13C, SLCO1B3, KLK2, TMEFF2, NROB1, PITX2, ACADL, SFRP4, AGR2, HNF11A, GRHL2, ou d'une quelconque combinaison correspondante est détecté.

5. Procédé selon la revendication 4, l'expression augmentée de KLK3, PGR, KCNN2, MYBPC1 et HOXB13 indiquant un risque élevé de cancer de la prostate.

6. Procédé selon l'une quelconque des revendications 1 à 3, le procédé comprenant en outre
la comparaison du niveau d'expression d'ARV7 de l'échantillon de sang complet du patient avec un niveau de référence d'expression d'ARV7, éventuellement le niveau d'expression d'ARV7 étant un niveau d'expression d'ARV7 dans un échantillon de sang complet provenant d'un individu sans cancer de la prostate ; et/ou
le fait de déterminer si le niveau d'expression d'ARV7 de l'échantillon de sang complet du patient est augmenté ou diminué par rapport au niveau de référence d'expression d'ARV7.

7. Procédé selon l'une quelconque des revendications 1 à 6, l'échantillon de sang complet étant collecté dans un tube de collecte de sang.

8. Utilisation d'une puce génique, comprenant des paires d'amorces et un panel de sondes configurées pour amplifier et détecter KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, GRHL2, ou une quelconque combinaison correspondante, pour le procédé selon l'une quelconque des revendications 1 à 7.

9. Utilisation selon la revendication 8, la puce génique comprenant des paires d'amorces et un panel de sondes configurées pour amplifier et détecter KLK3, ACADL, GRHL2, HOXB13, HSD3B1, TMP.ERG, ARV7, ARV567, FOLH1, KLK2, HSD3B2, AGR2, AZGP1, STEAP2, KCNN2, GPX8, TMEFF2, SPINK1, SFRP4, NROB1, FAM13C, HNF1A, CDH12, PGR, PITX2, MYBPC1, FOXA1, SRD5A2, COL1A1, NPY, UGT2B17, CLUL1, C9orf152, FLNC, GPR39, RELN, THBS2, CYP17A1, CYP3A5, BRS3, SNAI2, CDH12, NKX3.1, LGR5, TRPM8 et SLCO1B3.

10. Procédé d'identification d'un patient présentant un risque élevé de cancer de la prostate comprenant :
l'obtention d'ADNc d'un échantillon de sang complet du patient ;
la mise en contact de l'ADNc avec une puce génique, la puce génique comprenant un ensemble de paires d'amorces et un panel de sondes spécifiques pour des biomarqueurs d'ARNm indiquant un risque élevé de cancer de la prostate, les biomarqueurs d'ARNm étant choisis dans le groupe constitué par KLK3, PGR, KCNN2, MYBPC1, HOXB13, COL1A1, GPX8, FAM13C, SLCO1B3, KLK2, TMEFF2, NROB1, PITX2, ACADL, SFRP4, AGR2, HNF1A, et GRHL2 ;
et la mesure de niveaux d'expression de tous les biomarqueurs d'ARNm ; et
une augmentation du niveau d'expression d'au moins huit des biomarqueurs d'ARNm par comparaison avec un niveau de référence des au moins huit biomarqueurs d'ARNm étant mesurée.
